# EUROPEAN PATENT APPLICATION

(11) **EP 4 574 081 A1**
(43) Date of publication of application: **25.06.2025**
(21) Application number: 23315487.1
(22) Date of filing: 22.12.2023
(51) Int. Cl.: A61B 34/30, A61B 90/50

(54) **A MEDICAL INSTRUMENT HOLDER AND A MEDICAL SYSTEM**

(71) Applicant: Caranx Medical, 75013 Paris (FR)
(72) Inventor: TCHETCHE, Didier, 31000 Toulouse (FR); SMITS, Jonas Victor Harmen, 3360 Bierbeek (BE); SCHEGG, Pierre, 06000 Nice (FR)
(74) Representative: Hepp Wenger Ryffel AG

(57) **Abstract**

The invention relates to a medical instrument holder (101) configured for holding at least one medical instrument (11) in a translationally and/or rotationally movable manner, the medical instrument holder (101) comprising a frame unit (2) defining a guide path (21) for receiving and guiding the at least one medical instrument (11), a coupling unit (3) which is adapted to couple the frame unit (2) to an operating environment, and a plurality of fixation members (4) arranged along the guide path (21). The fixation members (4) are convertible between at least one inhibiting position for inhibiting or preventing translational and/or rotational movement of the medical instrument (11) and a release position for allowing translational and/or rotational movement of the medical instrument (11) relative to the frame unit (2).

## Description

The present invention relates to a medical instrument holder and a medical system configured for holding at least one medical instrument in a translationally and/or rotationally movable manner.

Medical procedures, in particular cardiac surgery such as transcatheter aortic valve implantation or replacement (TAVI/TAVR), typically require independent movability of medical instruments with respect to each other, e.g. for positioning and/or deploying a guidewire, a catheter, and/or an implant, in particular deploying a heart valve implant.

In particular, an exchange of medical instruments or a repositioning of medical instruments poses the risk of inadvertently repositioning other medical instruments. This is particularly evident in the case of medical procedures necessitating long medical instruments, e.g. during TAVI, with lengths up to 3 meters, where the exchange of a medical instruments such as a catheter while retaining a position/orientation of another medical instrument such as a guidewire requires two sterile operators for reliable operation of the medical instruments.

Malpositioning of an intracorporeal medical instrument has to be avoided since it poses severe risks of medical complications which may lead to tissue trauma, dislodgement of calcified material or clots leading to an occlusion, e.g. to a calcific embolism or thromboembolism, vascular dissection, and/or vascular hematoma or bleeding.

The operation of a variety of medical instruments, especially the movement of medical instruments relative to each other, can further lead to a variety of adverse surgical procedural difficulties, such as an incorrect placement/orientation of an implant or guidewire, e.g. placement of an intraventricular pacing wire. In addition, a suboptimal positioning/alignment of the medical instruments with respect to each other may result in longer procedure times, may require multiple operators for medical instruments which increases procedural costs, may demand more personal leading to staff shortages and costs, and/or may demand utmost vigilance from clinicians.

US2015173838 discloses a robotic system with an axial and rotational drive mechanism adapted to engage a percutaneous device with a control system which is adapted to engage and disengage the percutaneous device based on control signals.

However, the prior art lacks a solution which reliably and selectively modulates/prevents movement of a medical instrument, in particular a device which enables reliable movement of one medical instrument while modulating/preventing movement of another medical instrument.

It is an object of the present invention to overcome these and other disadvantages of the prior art and to provide a medical instrument holder and medical system which solves the above-mentioned technical problems. This object is solved by a medical instrument holder according to the independent claims. Further embodiments result from the dependent claims.

It is particularly an object of the present invention to streamline the medical procedures by diminishing the requisite proficiency and attention of the clinician when operating medical instruments, thereby providing a more cost-effective, simpler, and more expedient medical intraluminal procedure.

According to a first aspect of the invention, the medical instrument holder is configured for holding at least one medical instrument in a translationally and/or rotationally movable manner. The medical instrument holder comprises a frame unit defining a guide path for receiving and guiding the at least one medical instrument, a coupling unit which is adapted to couple the frame unit to an operating environment, and a plurality of fixation members. The fixation members are arranged along the guide path and are convertible between at least one inhibiting position for modulating or preventing translational and/or rotational movement of the medical instrument around or along a longitudinal axis of the medical instrument and/or around or along at least one axis distinct from the longitudinal axis and a release position for allowing translational and/or rotational movement of the medical instrument around or along the longitudinal axis and/or around or along the at least one axis distinct from the longitudinal axis relative to the frame unit.

The term longitudinal axis within the context of this application may relate to a curvilinear longitudinal axis, if e.g. the medical instrument is deflected to a bent shape.

For the purposes of this patent application, the term "modulating" shall be understood in its broadest sense to encompass any form of alteration, regulation, or adjustment of a function, activity, or process. This includes, but is not limited to, enhancing, upregulating, diminishing, suppressing, or inhibiting. In particular, inhibiting the movement may be particularly advantageous.

The fixation member in the release position may enable the medical instrument(s) to be removed/mounted laterally relative to the guide path from the fixation member or mounted on the fixation member. This may allow for simple arrangement/removal of the medical instrument(s) within the guide path.

The plurality of fixation members may be arranged over a distance along a trajectory of the guide path between 25 cm and 300 cm, in particular between 55 cm and 160 cm, preferably between 80 cm and 125 cm.

This enables reliably modulating/preventing translational and/or rotational movement of the medical instrument(s), in particular of a plurality of medical instruments simultaneously, which have a sufficient length for endoluminal procedures such as percutaneous coronary interventions. The medical instrument(s) are typically advanced and retracted such that only subsections are arranged within the guide path. A long guide path, in particular having a larger longitudinal length than the medical instrument, enables engaging the medical instruments at a wider range of procedural positions of the medical instruments.

Neighbouring fixation members may be spaced apart at least 7 cm, in particular at least 9 cm, preferably at least 10 cm, from each other along the guide path.

This allows the fixation members to engage the medical instrument(s) at a plurality of separate position of the guide path to modulate/prevent movement. At the same time these distances between fixation members are large enough to reduce the number of fixation members required and thus the costs. Moreover, this simplifies operation of the fixation members, i.e. conversion between the inhibiting and release position.

To further reduce the costs and operability associated with fixation members, the fixation members may be spaced apart event further apart from each other, in particular at least 15 cm, in particular 22.5 cm, preferably at least 30 cm.

The fixation members may be equidistantly arranged along the guide path. However, in an alternative embodiment, the fixation members are arranged non-equidistantly from each other for supporting procedural efficiency. For example, more fixation members may be placed at guide path positions corresponding to anatomical intraluminal positions of interest accessed by the medical instrument(s), e.g. the cardiac region accessed via the femoral artery. Other regions, e.g. the abdominal/thoracic aorta, may require fewer fixation members since they are less demanding to traverse.

The medical instrument holder may comprise at least three, in particular at least four, preferably at least five fixation members.

This facilitates modulating/preventing movement of the medical instrument(s) at a plurality of positions along the guide path effectively without overburdening the clinicians.

The at least one fixation member may be adapted to be movable along the guide path, in particular along a guide rail of the guide path. The at least one fixation member may be reversibly convertible between a locked state in which the fixation member is immovable with respect to the frame unit and an unlocked state in which the fixation member is movable relative to the frame unit.

This allows to customize of a position of the at least one fixation member to provide enhanced precision at the position at which it is most required for procedural efficiency. Furthermore, this provides a more versatile medical instrument holder which can easily be adapted for performing different medical procedures.

The at least one fixation member in the unlocked state may be movable along the guide path manually.

The at least one fixation member in the unlocked state may be configured to be coupled with the movement of the medical instrument along the guide path, such that the at least one fixation member in the inhibiting position may be moved together with the medical instrument.

The medical instrument holder may comprise an actuator for at least one fixation member. The actuator is configured for translationally and/or rotationally moving the medical instrument relative to the frame unit around or along the longitudinal axis and/or around or along the at least one axis distinct from the longitudinal axis by moving the fixation member when it is in the at least one inhibiting position.

The actuator may be adapted for moving the fixation member in the unlocked state along the guide path, in particular in a partially or fully automated manner. The actuator or another actuator of the medical instrument holder may be adapted for converting the fixation member between the inhibiting position and the release position.

The actuator for moving the fixation member in the unlocked state may be adapted to move the fixation member along the guide path with the medical instrument in a synchronized/unison manner while the fixation member is in the inhibiting position.

The at least one fixation member in a first inhibiting position may be adapted to modulate or prevent translational and/or rotational movement of a first medical instrument having a first cross-sectional dimension. In a second inhibiting position the at least one fixation member can be adapted to modulate or pre-vent translational and/or rotational movement of a second medical instrument having a second cross-sectional dimension. The first and second cross-sectional dimensions may be different from each other.

A plurality of inhibiting positions enables the at least one fixation member to modulate/prevent movement of a plurality of medical instruments selectively to facilitate medical percutaneous procedures.

The fixation member in the first inhibiting position may be adapted to enable translational and/or rotational movement of the second medical instrument, e.g. having a smaller cross-sectional dimension than the first medical instrument.

The fixation member in the second inhibiting position may be adapted to enable translational and/or rotational movement of the first medical instrument, e.g. having a larger cross-sectional dimension than the second medical instrument.

Alternatively, the fixation member in the first inhibiting position, the second inhibiting position, or a third inhibiting position may be adapted to prevent/inhibit translational and/or rotational movement of the first and the second medical instrument.

This may allow for selective individual actuation of different medical instruments by using different inhibiting positions of the fixation member.

However, in a preferred embodiment, the fixation member in the inhibiting position may be adapted to modulate/prevent movement of medical instruments of different cross-sectional dimensions simultaneously, in particular cross-sectional dimensions between 0.36 mm and 8 mm. This allows to engage guidewires having a diameter between 0.36 mm and 0.89 mm and a catheter having a diameter between 4.67 mm and 8 mm at the same time in the same inhibiting position.

The at least one fixation member, in particular all of the fixation members, of the plurality of fixation members may be selected from at least one of (i) a spring-loaded fixation member, (ii) a snap fit fixation member, (iii) a monolithically formed fixation member, or (iv) a fixation member which is convertible between the at least one inhibiting position and the release position, in particular convertible in a continuous and seamless manner, via an actuator of the medical instrument holder, and (v) a fixation member which is formed by a clamp or a plurality of clamps.

These fixation members facilitate simple conversion between the inhibiting and release position in a compact design.

The spring-loaded fixation member may be adapted to be actuatable via a spring-loaded mechanism between the inhibiting position and the release position. The spring loaded mechanism may additionally or alternatively be adapted to apply a frictional force to the received medical instrument in the inhibiting position, e.g. similar to a spring-loaded clothespin.

A monolithic fixation member according to (iii), in particular made from a polymeric plastic material, preferably a thermoplastic material, may further reduce material and assembly costs, have fewer potential points of failure, and provide a simple design.

A seamless conversion according to (iv) may allow for optimizing the force exerted on the medical instrument, e.g. such that differently sized medical instruments can be engaged reliably with the same force.

In a preferred embodiment the fixation member is a monolithically formed snap fit fixation member. The snap fit fixation member may comprise a cantilever spring for engaging the medical instrument(s).

The fixation member may be adapted such that the snap fit connection is closed in the inhibiting position and opened in the release position. The medical instrument(s) may be laterally mountable in the fixation member in the release position.

The snap fit fixation member may be adapted to be securely closed automatically by moving the medical instrument into the guide path.

The at least one fixation member, in particular all of the plurality of fixation members may be a fixation member which is adapted for modulating or preventing translational and/or rotational movement of a plurality of medical instruments which are arranged (i) parallel with respect to each other and the guide path or (ii) nested within each other and have different cross-sectional dimensions.

Modulating/preventing the movement of the plurality of nested and/or parallel medical instruments facilitates operation of the medical instruments in complex medical procedures such as vascular access according to the Seldinger method or an adapted Seldinger method and subsequent cardiac medical procedures.

The fixation member may comprise a plurality of fixation elements which are arranged in a row such that each fixation element is adapted to engage different medical instrument which are arranged in parallel.

The coupling unit may comprise a connecting element configured to be connected to an operating environment, in particular an operating table, or a frame unit positioner for moving the frame unit and the plurality of fixation members in at least one degree of freedom, in particular in two degrees of freedom, preferably in three degrees of freedom, with respect to the operating environment.

The frame unit positioner may be a robotic arm or a gravity-balanced arm operable by a user or a computer program. The gravity-balanced arm comprises a mechanism which counteracts the effects of gravity exerted on the gravity-balanced arm, the frame unit, and the fixation members, in particular without external actuation, such that a clinician can precisely position the frame unit in a smooth manner to different gravity-balanced positions.

The medical instrument holder, in particular a control unit of the medical instrument holder, may comprise the computer program adapted for operating the frame unit positioner. The medical instrument holder may comprise preprogrammed safety features such as a speed or motion limiters, positional feedback or auto-corrections which are adapted interact with the computer program.

The frame unit positioner and/or the control unit may comprise at least one electronic component for interacting with the computer program. The at least one electronic component may be selected from force sensors, accelerometers, gyroscopes, a collision avoidance system, and/or a user safety interface adapted for visual, auditory, and/or haptic feedback, e.g. to alert to potential safety features.

The safety features and/or the electronic component may be selectively activatable by a user/clinician via a user interface of the frame unit positioner.

The medical instrument holder may have a connection port, in particular formed by a fixation member, which is arranged at a distal end of the guide path and configured for connecting a percutaneous introducer assembly which is adapted for percutaneous introduction of a medical instrument into a body duct of a patient.

This simplifies a modular use of the medical instrument holder with a percutaneous introducer assembly and ensures an optimized operability/alignability of the guide path with the introducer assembly.

A medical system comprising the medical instrument holder may comprise a percutaneous introducer assembly or system comprising the percutaneous introducer assembly connectable to the connection port. The introducer assembly or system may be adapted for carrying out a Selinger technique or an adapted Seldinger technique, preferably in a partially or fully automated manner. The system and/or introducer assembly of the medical system, may be formed as disclosed in the pending international patent application PCT/EP2023/061428 filed on 1 May 2023 with the title "A Biomedical device, a system comprising a biomedical device, a method for percutaneous catheterization of a body duct, and a method for aligning an external imaging means" which is incorporated herein by reference.

The medical instrument holder may comprise a storage unit. The storage unit may be configured for storing and feeding at least one medical instrument to the guide path and/or receiving at least one medical instrument from the guide path, in particular in a sterile manner.

The storage unit facilitates the usage of different medical instruments and simplifies changing/storing the medical instruments, in particular idle medical instruments, e.g. differently shaped guidewires which are used during complex medical procedures.

The frame unit may comprise a loading member which is configured to frictionally engage the at least one medical instrument and move, in particular translationally move, the medical instrument into the guide path or remove the medical instrument from the guide path. Alternatively, the loading member may be configured to engage the storage unit and translationally connect/disconnect the storage unit for feeding/receiving the at least one medical instrument.

This loading member facilitates positioning of the medical instruments in the guide path and changing medical instruments in a reliable and precise manner allowing for a time saving procedure such that clinicians can focus on patient care.

The storage unit and/or the frame unit, in particular the guide path, can be (i) deflectable from an at least partially straight shape such that the storage unit and/or the guide path have a deflected geometric shape and/or (ii) formed such that the storage unit and/or the guide path define the deflected geometric shape. The geometric shape may be an at least partial U-shape, a circular shape, or a spiral shape.

This reduces the dimensions of the storage unit and/or the frame unit rendering it more portable and providing increased working space for clinicians and other medical instruments, e.g. by occupying less space on the operating table.

The guide path may be partially or completely circumferentially closed, in particular circumferentially closed along the entire guide path, for receiving the at least one medical instrument, in particular a plurality of medical instruments. Alternatively, the guide path may be partially or completely circumferentially open, preferably circumferentially open along the entire guide path.

A completely closed guide path is easier to maintain in a sterile environment and provides sufficient guidance for the medical instrument(s) between the fixation members.

An at least partially circumferentially open guide path ensures that the at least one medical instrument, in particular the plurality of medical instruments are radially accessible by a clinician which facilitates manual/robotic operation of the medical instruments.

In one embodiment, the medical instrument holder is configured such that only the fixation members are arranged to radially engage and confine the medical instrument(s) in the guide path. This improves usability by a clinician by rendering the medical instrument(s) accessible along their entire extracorporeal longitudinal section and thereby simplifies translational/rotational movement of the medical instrument(s).

The guide path may be adapted to receive the plurality of medical instruments in an at least partially nested or parallel manner.

The medical instrument holder may comprise a cover, in particular a drape, which is arranged and configured for covering the frame unit and/or the coupling unit, preferably in a sterile manner.

This enables to arrange the medical instrument holder, in particular the frame unit and/or the coupling unit, in a sterile operating environment.

Sections of the medical instrument holder such as the coupling unit may not necessarily be kept sterile.

The fixation member may comprise or consist of a silicone rubber, an acrylonitrile butadiene styrene, a thermoplastic elastomer, a polyurethane, in particular a polyurethane foam, a medical-grade polyvinyl chloride, a natural rubber, a polyethylene, in particular a polyethylene foam, and/or polypropylene.

These materials provide a sufficient flexibility which is advantageous for modulating or preventing the movement of the medical instrument.

The fixation member, in particular a monolithic fixation member, may be manufactured by an additive manufacturing process. This fixation member may comprise or consist of thermoplastic polyurethane, polyethylene terephthalate glycol, polyamide, thermoplastic elastomers, thermoplastic copolyester, resins, and/or polypropylene.

The fixation member may be adapted for single-use applications, in particular by comprising or consisting of polypropylene and/or low-density polyethylene, or for repeated-use applications, in particular by comprising or consisting of a durable thermoplastic elastomer or a high-density polyethylene.

Another aspect of the invention relates to a medical system comprising the previously described medical instrument holder and at least one medical instrument, in particular a catheter and/or a guidewire, which has a longitudinal length between 70 cm and 350 cm, in particular between 80 cm and 200 cm, preferably between 85 cm and 125 cm.

This ensures that the longitudinal length of the medical instrument(s) is sufficient for carrying out complex medical intraluminal procedures, in particular TAVI/TAVR.

The medical instrument(s), in particular at least one guidewire and at least one catheter, preferably a catheter for deploying a heart valve prosthesis, of the medical system may be adapted for cardiac surgery, in particular deploying the heart valve prosthesis, preferably during TAVI/TAVR. The guidewire may have a pre-shaped bent distal tip and be adapted to be deployed within the left ventricle of the heart.

The frame unit of the medical system may have at least three fixation members which are sequentially arranged along the guide path over at least 50%, in particular at least 75%, preferably at least 100%, of the longitudinal length of the at least one medical instrument.

This ensures that the fixation members are arranged in a manner sufficient to reliably engage the medical instrument(s) sequentially at a plurality of separate positions of the guide path to modulate/prevent movement if required. Moreover, this ensures that the medical instrument(s) in the guide path are assessable by a fixation member irrespective of the position relative to the frame unit, e.g. if the medical instrument(s) were introduced partially in an intraluminal procedure.

Fixation members which are sequentially arranged along the guide path over at least 100% of the longitudinal length of the at least one outer medical instrument allow to modulate/prevent movement of an inner nested medical instrument with at least one fixation member while the outer medical instrument enclosing the inner nested instrument can be advanced/retracted.

The invention is now described with reference to certain embodiments and figures which show:
Figure 1: a schematic representation of a medical system comprising a medical instrument holder which has a plurality of fixation members,
Figures 2A - 2D: a schematic cross-sectional view of the medical instrument holder used for removing an intraluminal catheter from a guidewire,
Figures 3A - 3D: a schematic cross-sectional view of the medical instrument holder used for placing an intraluminal catheter over a guidewire,
Figure 4A: a perspective side view of a medical instrument holder comprising a plurality of fixation members,
Figures 4B and 4C: a fixation member formed by a monolithic clamp of Fig. 4A in an inhibiting position and in a release position,
Figure 4D: a frame unit positioner of the medical instrument holder for moving the frame unit.

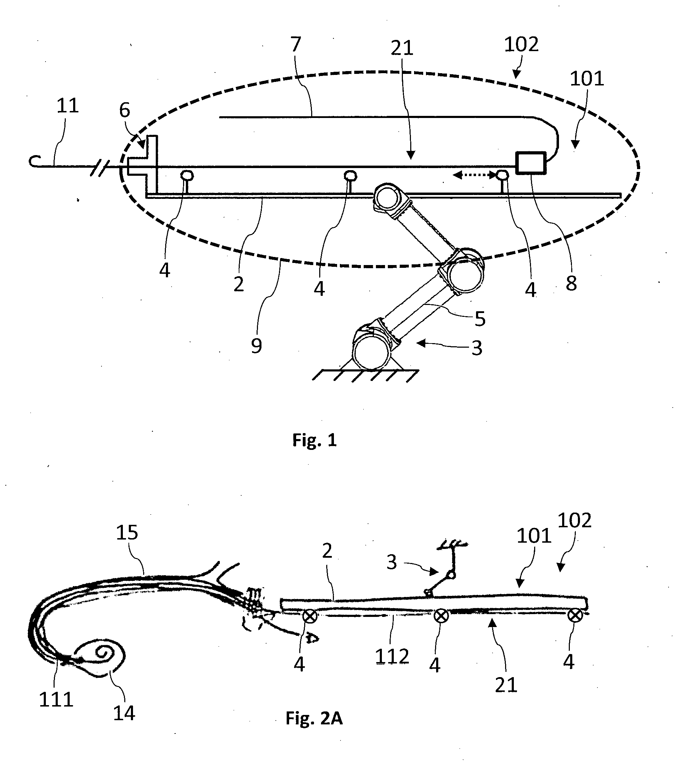

Figure 1 shows a schematic representation of a medical system 102 comprising a medical instrument holder 101 which has a plurality of fixation members 4. The fixation members 4 are convertible between an inhibiting position for modulating/preventing translational and rotational movement of a medical instrument 11 and a release position for allowing translational/rotational movement of the medical instrument 11. The medical instrument 11 is arranged along a guide path 21 of a frame unit 2.

The frame unit 2 of the medical instrument holder 101 has a longitudinal length of 165 cm. The frame unit 2 is formed by a guide rail and is positioned such that three fixation members 4 attached to the frame unit can engage the medical instrument 11 placed in the guide path 21. Figure 1 shows that the three fixation members 4 are spaced apart from each other by 45 cm such that the medical instrument 11 can be engaged at spaced apart longitudinal positions in the guide path 21. This allows medical instruments 11 which are translationally moved partially out of the guide path 21, e.g. during intraluminal insertion or removal to be engaged by at least one fixation member 4.

The fixation members 4 have a locked state in which the fixation member 4 is fixed relative to the frame unit 2 and an unlocked state in which the fixation member 4 can be moved along the frame unit 2 indicated by the dashed two-headed arrow. The fixation member 4 in Fig. 1 may have a quick-release lever for converting the fixation members 4 between the locked and unlocked state which allows a simple and efficient manual movement of the fixation member 4 along the guide rail and locking of the fixation members 4 at a specific position of the guide rail.

Figure 1 further shows a storage unit 7 formed by a channel for receiving the medical instrument 11 from the guide path 21. The storage unit 7 has a U-shape and is connected to a proximal end of the guide path 21 such that at least one medical instrument 11 can be fed to the guide path 21 or received from the guide path 21. The medical instrument holder 101 in Fig. 1 has a loading member 8 which is adapted for feeding the medical instrument 11 from the storage unit 7 into the guide path 21 and remove the medical instrument 11 from the guide path 21 by moving it into the storage unit 7. The loading member 8 can comprise an actuator having two rotational rollers for engaging and feeding/removing the medical instrument 11 by radially engaging the medical instrument 11.

The storage unit 7 is disconnectable from the guide path 21 and preferably allows for sterile storage of the medical instrument 11 within the storage unit 7. The medical system 102 may comprise a plurality of storage units 7 which are connectible/disconnectable to/from the guide path 21 for receiving and feeding medical instruments 11. This may facilitate exchanging and storing medical instruments 11.

The frame unit 2 is connected to the operating environment via a coupling unit 3. The coupling unit 3 has a frame unit positioner 5 formed by a gravity-balanced arm which may be placed on an operating table in proximity of the operating environment. The gravity-balanced arm is adapted to position the frame unit 5 in three degrees of freedom.

The frame unit positioner 5 allows for a rotational movement of the frame unit 2 along two rotational degrees of freedom, i.e. a pitch and a yaw movement. The pitch movement may be carried out by rotating the frame unit 2 via the frame unit positioner 5 to adjust the inclination of the frame unit 2 relative a base of an operating table. The yaw movement may be carried out by rotating the frame unit 5 around a base of the frame unit positioner 5 in a plane parallel to the operating table.

At a distal end from a clinician's perspective, the medical instrument holder 101 has a connection port 6. The connection port 6 is adapted for connecting a percutaneous introducer assembly for percutaneous introduction of a medical instrument such as a sheath, a catheter, a dilator, a trocar, an implant, and/or a guidewire into a body duct, in particular the artery, of a patient. The connection port 6 may be adapted for connecting a hub, e.g. of a sheath, of an introducer assembly, such that using the medical instrument holder 101 after or during the Seldinger or adapted Seldinger techniques is facilitated.

Figure 1 further shows that a cover 9 formed by a drape covers the medical instrument holder 101 such that the medical instrument(s) 11, the storage unit 7, the frame unit 2, the loading member 8, and fixation members 4 are arranged in a sterile environment.

The cover, in particular the drape, can have a longitudinal elongated sealing structure such as a slide fastener to render the guide path at least partially sterile but easily assessable. This may allow the medical instrument(s) 11 to be arranged/aligned in/along the guide path 21 within the cover.

Figures 2A - 2D show a schematic cross-sectional view of the medical instrument holder 101 used for removing an intraluminal catheter 111 from a guidewire 112. The medical instrument holder 101 comprises a frame unit 2 which is connected via a coupling unit 3, e.g. comprising a gravity-balanced arm, to a solid support such as an operating table. The frame unit 2 defines a guide path 21 parallel to the frame unit 2 which is adapted for receiving medical instruments 111, 112, in particular in parallel to each other or in a nested manner. The guide path 21 may be configured to be laterally accessible, e.g. manually or robotically laterally accessible, for manipulation of the medical instruments 111, 112. The medical instruments 111, 112 in Figs. 2A - 2D are only retained within the guide path 21 by the fixation members 4. The fixation members 4 in Figs. 2A - 2D are formed by monolithically formed snap fit fixation members which allow lateral movement of the medical instruments 111, 112 into or away from the guide path 21 in a release position of the fixation members 4 and inhibit translational and/or rotational movement of the medical instruments 111, 112 while the fixation members 4 are in the inhibiting position. The clinician is still able to translationally/rotationally move the medical instruments 111, 112 while the fixation members 4 are in the inhibiting position. However, the fixation members 4 modulate, in particular inhibit, the movement such that accidental movement is prevented while providing haptic mechanical feedback for a clinician when moving the medical instruments 111, 112 which facilitates manual positioning or prevents slippage for motorized manipulation solutions. The manual or motorized manipulation of the medical instruments 111, 112 is illustrated in Figs. 2A - 2D by a schematic representation of a hand.

The medical instrument holder 101 or system 102 can have at least one actuator for moving the medical instrument(s) 111, 112, in particular by moving the fixation members 4. In this manner, the fixation members 4 can simultaneously serve as a fixation member for multiple medical instruments 111, 112 while also facilitating positioning of the medical instruments 111, 112 along the guide path 21 via the actuators.

The medical instrument holder 101 or system 102 can have at least one actuator for converting the fixation members 4 between the inhibiting and the release position, in particular in addition to the previously mentioned actuator for moving the fixation members 4. This may allow to selectively grip the medical instrument(s) 111, 112 when the fixation members 4 are converted from the release position to the inhibiting position and longitudinally move the medical instrument(s) 111, 112.

Alternatively, the fixation members 4 may be independently manually convertible between a locked state and an unlocked state for repositioning, e.g. via a setting screw attaching it to the guide rail of the guide unit 2 or a motor and a cam for converting the fixation members between the locked and unlocked state.

Even though Figs. 2A - 2D only show three fixation members 4, in a preferred embodiment, the medical instrument holder 101 may have at least four or five fixation members 4.

Figure 2A shows that the placement of the catheter 111 and the guidewire 112, e.g. a Safari guidewire 112, which has a distal tip that exhibits a preformed curve for deployment in the left ventricle of the heart during a TAVI/TAVR procedure. The representation of the hand in figure 2A further schematically illustrates that the catheter 111 can be retracted after being intraluminally, percutaneously inserted through the aorta 15 and aortic arch of a patient up to the heart 14.

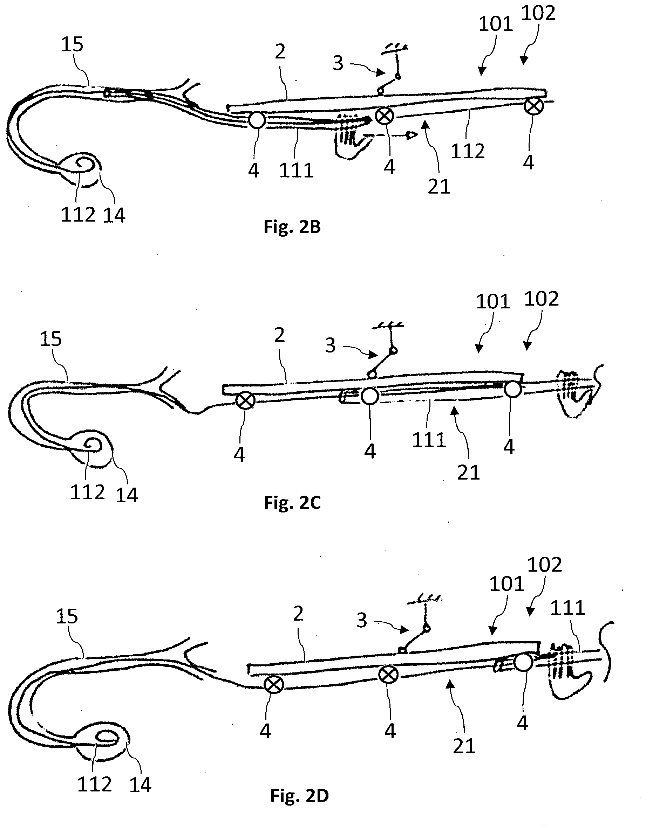

The distal-most fixation member 4 is in a release position in Fig. 2B such that the catheter 111 can be secured in the guide path 21 and guided across the fixation member 4 without inhibiting movement of the catheter 111. This fixation member 4 is preferably adapted such that the catheter 111 can be guided through the fixation member 4 in the release position. However, the other fixation members 4 are still in an inhibiting position indicated by the "X" marking such that movement of the guidewire 112 can still be prevented/inhibited while retracting the catheter 111. The catheter 111 has not yet reached the position of these fixation members 4.

Figure 2C and 2D show that the catheter 111 is proximally retracted further along the guide path 21 and the other fixation members 4 are converted to the release position such that the catheter 111 can be easily removed from a proximal end of the medical instrument holder 101. Once a distal tip of the catheter 111 is moved across the distal-most fixation member 4, the distal-most fixation member 4 can be converted to the inhibiting position again indicated by the "X" marking, such that the guidewire 112 is reliably prevented from accidental translational/rotational movement. The total distance between all the fixation members 4 is preferably larger than the length of the medical instrument 111, 112, in particular the catheter 111 and/or guidewire 112 so that one fixation member 4 always can inhibit movement of the guidewire 112 while not inhibiting movement of the catheter 111. In addition, too many fixation members 4 increase the workload of a clinician if they are manually converted while too few fixation members 4 do not allow for reliable fixation of all medical instruments 111, 112. Three to five fixation members proved to be a reliable compromise. This allows that the catheter 111 can be proximally retracted while at least one fixation member 4 is in the inhibiting position to keep e.g. the guidewire 112 in a specific longitudinal position/orientation within the patient without requiring multiple medical instrument operators or clinicians for the procedure.

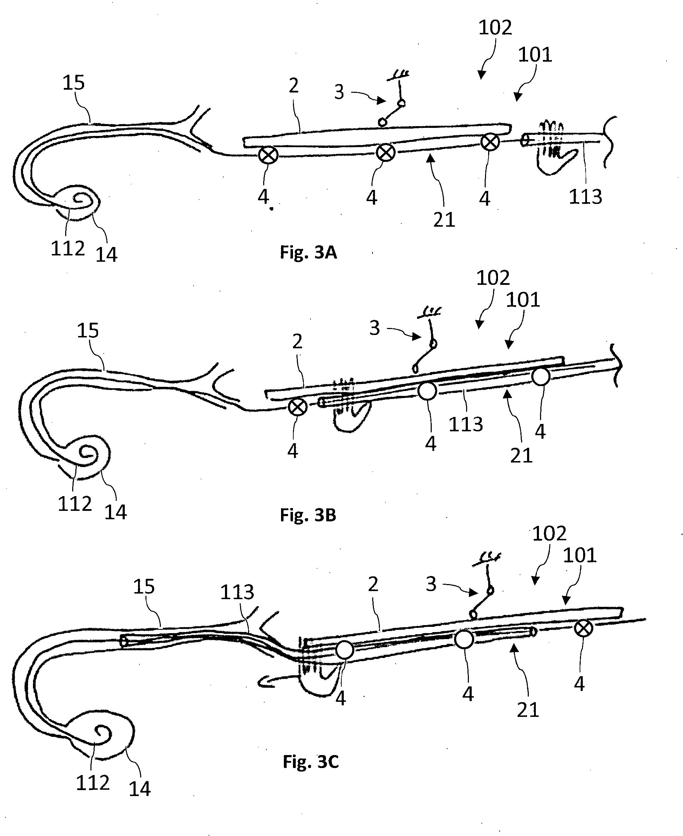

Figures 3A - 3D show a schematic cross-sectional view of the medical instrument holder 101 shown in Figs. 2A - 2D which is used for placing an intraluminal catheter 113 and guidewire 112. The medical instrument holder 101 and the guidewire 112 of Figs. 3A - 3D are formed as described in Figs. 2A - 2D. However, the catheter 113 shown in Fig. 3A - 3D may be adapted for deploying an aortic heart valve prosthesis in the aortic annulus of a patient. This catheter 113 can be intralumnially introduced after successful navigation of the guidewire 112 to the left ventricle and deployment of the guidewire with a navigational catheter 113 (e.g. via a catheter of Figs. 2A - 2D). This exemplary illustrates that the exchange of medical instruments 112, 113 can be facilitated and inadvertently moving other medical instruments such as the guidewire 112 can be avoided.

Figure 3A shows that the curved distal tip of the guidewire 112 in Fig. 3A was advanced through the aorta 15 and deployed in the left ventricle of the heart 14. Figure 3A further shows that the catheter 113 is advanced in a distal direction over the guidewire 112 along the guide path 21 in a nested manner. The coupling unit 3 is adapted to adjust the frame unit 2 in multiple degrees of freedom to facilitate percutaneous access of the medical instruments 112, 113.

Figure 3B shows that the first two proximal fixation members 4 were converted to the release position to enable advancement of the catheter 113 in the distal direction. However, the distal-most fixation member 4 is still in the inhibiting position to prevent inadvertently moving the guidewire 112 such that the guidewire 112 reliably remains in the desired position/orientation within the left ventricle.

Figure 3C shows that the catheter 113 can be advanced further along the guide path 21. For advancing the catheter 113 the two distal fixation members 4 were converted to the release position and instead the proximal most fixation member 4 is converted to the locking position such that a position/orientation of the guidewire 112 is locked at any time during the advancement. For this reason, the distance of the fixation members 4 along the guide path 21 is preferably longer than a longitudinal length of the medical instruments 112, 113.

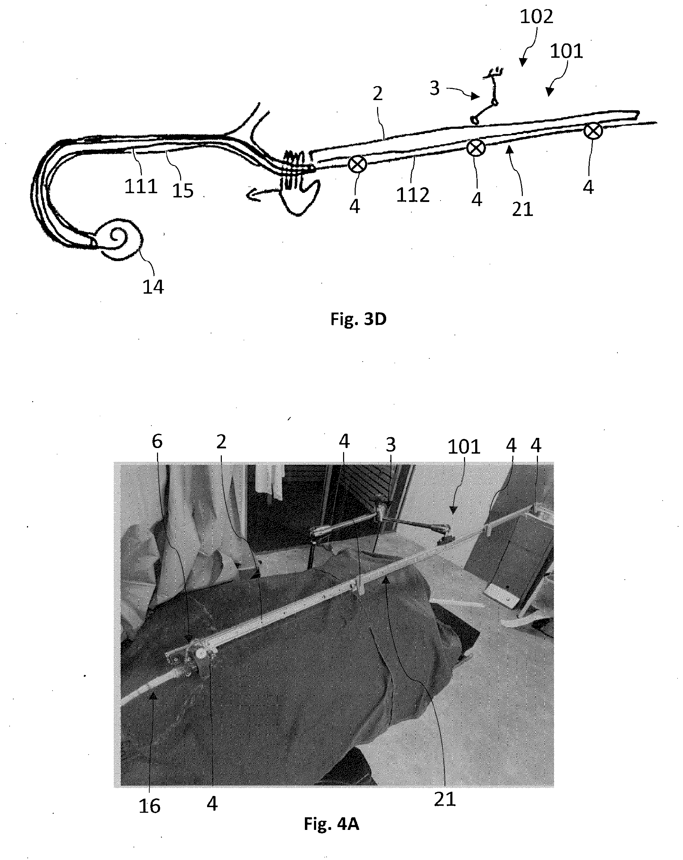

Figure 3D shows that the catheter 113 is completely advanced through the aorta 15 and the aortic arch up to the heart 14. The catheter 113 is adapted for deploying a heart valve prosthesis in the aortic annulus in a manner known to the person skilled in the art, i.e. via a balloon catheter or via a self-expandable heart valve prosthesis (not shown in Figs. 3A - 3D).

Alternatively, the fixation members 4 in Figs. 2A - 2D and 3A -3D may be convertible between the inhibiting and release position via an actuator of the medical instrument holder 101. The medical instrument holder 101 may be adapted to partially or fully automatedly convert the fixations members 4. The conversion of the fixation members 4 may be based on an input of a proximity sensor which detects the proximity of a medical instrument upon retraction/advancement according to the previously described procedure. This may further reduce the workload for clinicians and streamline the procedure.

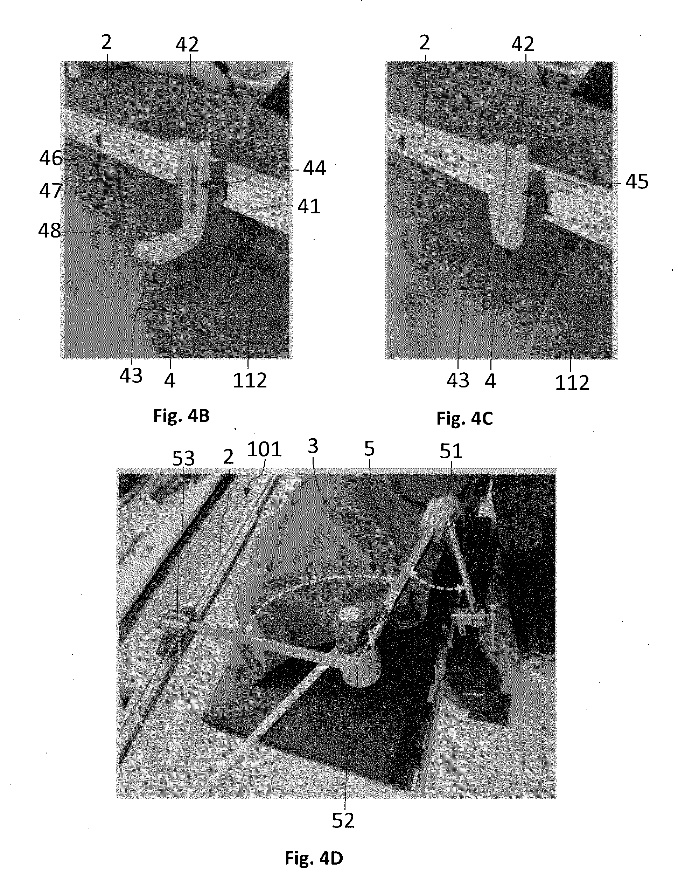

Figure 4A shows a perspective side view of a medical instrument holder 101 comprising a plurality of fixation members 4. The medical instrument holder 101 has a frame unit 2 formed by a guide rail which has a longitudinal length of 180 cm which defines a guide path 21 parallel to the frame unit 2. The medical instrument holder 101 has four fixation members 4 which are located along the guide path 21 and equidistantly spaced apart from each other. The distal-most fixation member 4 further forms a connection port 6 which is adapted for connecting an introducer assembly 16, in particular an introducer assembly for percutaneous catheterization, e.g. of the femoral artery, e.g. shown in PCT/EP2023/061428. The frame unit 2 is connected to an operating table via a coupling unit 3 (see Fig. 4D). The fixation members 4 are formed by monolithically formed clamps which are adapted to be converted from a release position to the inhibiting position via a snap fit mechanism (see Figs. 4B and 4C) .

Figures 4B and 4C show a fixation member 4 formed by the monolithic clamp of Fig. 4A in an inhibiting position and in a release position respectively. The fixation member 4 is preferably manufactured by injection molding, additive procedures, or compression molding. The fixation members 4 have a hinge 41 which is centrally arranged on the fixation member 4 such that two parts of fixation member 4 can be pivoted relative to each other to be converted between a release position 44 (see Fig. 4B) and an inhibiting position 45 (see Fig. 4C).

The fixation member 4 comprises a recess 42 on one end of the fixation member 4 and a protrusion 43 arranged on the other end of the fixation member 4. When the fixation member 4 is converted from the release position 44 in which the recess 42 and protrusion 43 are spaced apart to the inhibiting position 45, the recess 42 and protrusion 43 are brought into contact and engage each other to form a snap-fit connection. This snap-fit connection may be used for releasably securing the medical instrument(s) 112 between the two parts of the fixation member 4 in the inhibiting position 45.

In the release position 44 in Fig. 4B, a medical instrument such as a guidewire 112 can be laterally secured in the fixation member 4. In the inhibiting position 45 in Fig. 4C, the medical instrument 112 is engaged from two opposing sides via three engagement elements 46, 47, 48. At least one of the engagement elements 46, 47, 48 may define an engagement element which is only connected to the fixation member at its ends. The at least one of the engagement elements 46, 47, 48 is adapted to exert a spring tension force on the medical instrument(s) in the inhibiting position 45 by forming a cantilever spring or a fixed-fixed beam spring. This further enables multiple medical instruments 112 to be received simultaneously by the fixation member 4 in the inhibiting position 45 and facilitates operation of the fixation member 4.

Figure 4D shows a frame unit positioner 5 of the medical instrument holder 101 for moving the frame unit 2. The frame unit positioner 5 in Fig. 4D has three joints which allow rotational movement of links of the frame unit positioner 5 relative to each other indicated by the dashed arrows. This enables reliable positioning of a frame unit 2 for holding the medical instrument(s) in a percutaneous medical procedure.

## Claims

1. A medical instrument holder (101) configured for holding at least one medical instrument (11) in a translationally and/or rotationally movable manner, the medical instrument holder (101) comprising:
- a frame unit (2) defining a guide path (21) for receiving and guiding the at least one medical instrument (11),
- a coupling unit (3) which is adapted to couple the frame unit (2) to an operating environment, and
- a plurality of fixation members (4) arranged along the guide path (21) which are convertible between
at least one inhibiting position for modulating or preventing translational and/or rotational movement of the medical instrument (11) around or along a longitudinal axis of the medical instrument and/or around or along at least one axis distinct from the longitudinal axis, and
a release position for allowing translational and/or rotational movement around or along the longitudinal axis and/or around or along the at least one axis distinct from the longitudinal axis of the medical instrument (11) relative to the frame unit (2).

2. The medical instrument holder (101) according to claim 1, wherein the plurality of fixation members (4) is arranged over a distance along a trajectory of the guide path between 25 cm and 300 cm, in particular between 55 cm and 160 cm, preferably between 80 cm and 125 cm.

3. The medical instrument holder (101) according to one of the preceding claims, wherein neighbouring fixation members (4) are spaced apart at least 7 cm, in particular at least 9 cm, preferably at least 10 cm, from each other along the guide path (21).

4. The medical instrument holder (101) according to one of the preceding claims, wherein the medical instrument holder (101) comprises at least three, in particular at least four, preferably at least five fixation members (4).

5. The medical instrument holder (101) according to one of the preceding claims, wherein at least one fixation member (4) is adapted to be movable along the guide path (21), in particular along a guide rail of the guide path (21), wherein the at least one fixation member (4) is preferably reversibly convertible between a locked state in which the fixation member (4) is immovable with respect to the frame unit (2) and an unlocked state in which the fixation member (4) is movable relative to the frame unit (2).

6. The medical instrument holder (101) according to claim 5, wherein the instrument holder (101) comprises an actuator for at least one fixation member (4), wherein the actuator is configured for translationally and/or rotationally moving the medical instrument (11) relative to the frame unit (2) around or along the longitudinal axis of the medical instrument and/or around or along the at least one axis distinct from the longitudinal axis by moving the fixation member (4) when it is in the at least one inhibiting position.

7. The medical instrument holder (101) according to one of the preceding claims, wherein the at least one fixation member (4) in a first inhibiting position is adapted to modulate or prevent translational and/or rotational movement of a first medical instrument (11) having a first cross-sectional dimension and the at least one fixation member (4) in a second inhibiting position is adapted to modulate or prevent translational and/or rotational movement of a second medical instrument having a second cross-sectional dimension, wherein the first and second cross-sectional dimensions are different from each other.

8. The medical instrument holder (101) according to one of the preceding claims, wherein at least one fixation member (4), in particular all of the fixation members (4), of the plurality of fixation members (4) are selected from at least one of:
- A spring-loaded fixation member (4),
- A snap fit fixation member (4),
- A monolithically formed fixation member (4), preferably a monolithically formed snap fit fixation member (4),
- A fixation member (4) which is convertible via an actuator of the medical instrument holder (101) between the at least one inhibiting position and the release position, in particular convertible in a continuous and seamless manner, and
- A fixation member (4) which is formed by a clamp or a plurality of clamps.

9. The medical instrument holder (101) according to one of the preceding claims, wherein the at least one fixation member (4), in particular all of the plurality of fixation members (4), are selected from a fixation member (4) which is adapted for modulating or preventing translational and/or rotational movement of a plurality of medical instruments (11) which are arranged (i) parallel with respect to each other and the guide path (21) or (ii) nested within each other and have different cross-sectional dimensions.

10. The medical instrument holder (101) according to one of the preceding claims, wherein the coupling unit (3) comprises:
- A connecting element (31) configured to be connected to an operating environment, in particular an operating table, or
- A frame unit positioner (5), in particular a robotic arm or a gravity-balanced arm operable by a user or a computer program, for moving the frame unit (5) and the plurality of fixation members (4) in at least one degree of freedom, in particular in two degrees of freedom, preferably in at least three degrees of freedom, with respect to the operating environment.

11. The medical instrument holder (101) according to one of the preceding claims, wherein the medical instrument holder (101) has a connection port (6), in particular formed by a fixation member (4), which is arranged at a distal end of the guide path (21) and configured for connecting a percutaneous introducer assembly which is adapted for percutaneous introduction of a medical instrument (11) into a body duct of a patient.

12. The medical instrument holder (101) according to one of the preceding claims, wherein the medical instrument holder (101) comprises a storage unit (7), the storage unit (7) being configured for storing and feeding at least one medical instrument (11) to the guide path (21) and/or receiving at least one medical instrument (11) from the guide path (21), in particular in a sterile manner.

13. The medical instrument holder (101) according to one of the preceding claims, wherein the frame unit (2) comprises a loading member (8) which is configured to frictionally engage the at least one medical instrument (11) and move, in particular translationally move, the medical instrument (11) into the guide path (21) or remove the medical instrument (11) from the guide path (21) or
is configured to engage the storage unit (7) and translationally connect/disconnect the storage unit for feeding/receiving the at least one medical instrument (11).

14. The medical instrument holder (101) according to one of the preceding claims, wherein the storage unit (7) and/or or the frame unit (2), in particular the guide path (21), are one of:
- deflectable from an at least partially straight shape such that the storage unit (7) and/or the guide path (21) have a deflected geometric shape, in particular an at least partial U-shape, a circular shape, or a spiral shape, and/or
- formed such that the storage unit (7) and/or the guide path (21) define the deflected geometric shape, in particular the at least partial U-shape, the circular shape, or the spiral shape.

15. The medical instrument holder (101) according to one of the preceding claims, wherein the guide path (21) is one of
- a partially or completely circumferentially closed guide path (21), in particular along the entire guide path (21),
- a partially or completely circumferentially open guide path (21), preferably along the entire guide path (21) .

16. The medical instrument holder (101) according to one of the preceding claims, wherein the medical instrument holder (101) comprises a cover (9), in particular a drape, which is arranged and configured for covering the frame unit (2) and/or the coupling unit (3), preferably in a sterile manner.

17. A medical instrument holder (101) according to one of the preceding claims, wherein the fixation member comprises or consists of a silicone rubber, an acrylonitrile butadiene styrene, a thermoplastic elastomer, a polyurethane, in particular a polyurethane foam, a medical-grade polyvinyl chloride, a natural rubber, a polyethylene, in particular a polyethylene foam, and/or polypropylene.

18. A medical system (102) comprising the medical instrument holder (101) according to one of the preceding claims and at least one medical instrument (11), in particular a catheter and/or a guidewire, which has a longitudinal length between 70 cm and 350 cm, in particular between 80 cm and 200 cm, preferably between 85 cm and 125 cm.

19. The medical system (1,02) according to claim 16, wherein the frame unit (2) has at least three fixation members (4) which are sequentially arranged along the guide path (21) over at least 50%, in particular at least 75%, preferably at least 100%, of the longitudinal length of the at least one medical instrument (11).
